# EUROPEAN PATENT SPECIFICATION

(11) **EP 2 152 246 B1**
(45) Date of publication and mention of the grant of the patent: **22.09.2010**
(21) Application number: 08759444.6
(22) Date of filing: 07.05.2008
(51) Int. Cl.: A61K 9/20, A61K 33/06, A61K 35/02

(54) **SOLID COMPOSITION COMPRISING A CLAY SUCH AS KAOLIN**
FESTE ZUSAMMENSETZUNG MIT EINER TONERDE WIE ETWA KAOLIN
NOUVELLE COMPOSITION COMPRENANT UNE ARGILE COMME LE KAOLIN

(30) Priority: 09.05.2007 GB 0708929
(43) Date of publication of application: 17.02.2010
(73) Proprietor: GlaxoSmithKline Consumer Healthcare GmbH & Co.KG., 77815 Buehl (Baden) (DE)
(72) Inventor: CHRISTIAN, Stefanie, D-71070 Herrenberg (DE); HARKENTHAL, Michael, D-77815 Buehl (baden) (DE); SCHOBERT, Ulrich Thomas, D-71070 Herrenberg (DE)
(74) Representative: Sewell, Richard Charles
(86) International application number: PCT/EP2008/055589
(87) International publication number: WO 2008/138816

(56) References cited:
- EP-A- 1 747 775
- WO-A-2006/045152
- US-A- 5 660 860
- US-A1- 2004 009 246
- JAMINET F: "[RESEARCH ON THE ACTION OF ADJUVANTS OF TABLETS ON CERTAIN CHARACTERISTICS OF THE LATTER. I. INFLUENCE OF THE VISCOSITY OF SODIUM CARBOXYMETHYLCELLUSOSES USED AS BINDERS AND AS DISINTEGRATORS ON THE SPEED OF DISINTEGRATION OF KAOLIN TABLETS.]" JOURNAL DE PHARMACIE DE BELGIQUE 1964 MAR-APR, vol. 19, March 1964 (1964-03), pages 144-150, XP009122030 ISSN: 0047-2166

## Description

The present invention relates to a therapeutic composition comprising kaolin, at least one disintegrant, at least one lubricant and at least one binding agent, and in particular to a palatable clay composition in the form of a tablet, and to a process for the preparation of the therapeutic composition.

Clay compositions, for example comprising a natural clay such as healing earth or kaolin have been known since ancient times. Healing earth, comprising a range of minerals and trace elements, has been used for treating many ailments and disorders of the body. Healing earth is available commercially as a powder for external application, such as the powder preparation, Heilerde, sold under the brand name Bullrich. It is also sold in capsule form for internal application, under the Bullrich brand.

Kaolin, another natural clay, has been used as an adsorbent for external cleansing of the skin and is also known for internal cleansing of the body. Kaolin is also known as *Bolus alba,* china clay, porcelain clay, white bole, and argilla and has the approximate chemical formula of H₂Al₂Si₂O₈ (H₂O). Finely divided particles of kaolin yield a very large surface area and are known to adsorb a wide variety of materials. For internal cleansing of the body, to be effective therapeutically, kaolin is normally administered at a dose of from about 2 to about 20g per day. For external cleansing of the body, to be effective therapeutically, a dose of from 2 to about 80g per day may be administered. For example, a dose of from about 2 to about 20g may be administered, in an amount from one to four applications daily, depending on the nature and amount of external cleansing required, for example the area of skin to be cleansed. When taken in capsule form, a daily dose may require a large number of capsules to be consumed, for example a daily dose of the capsules sold under the Bullrich brand consists of six capsules.

JAMINET F: "[RESEARCH ON THE ACTION OF ADJUVANTS OF TABLETS ON CERTAIN CHARACTERISTICS OF THE LATTER. I. INFLUENCE OF THE VISCOSITY OF SODIUM CARBOXYMETHYLCELLUSOSES USED AS BINDERS AND AS DISINTEGRATORS ON THE SPEED OF DISINTEGRATION OF KAOLIN TABLETS.]" JOURNAL DE PHARMACIE DE BELGIQUE 1964 MAR-APR, vol. 19, March 1964 (1964-03) , pages 144-150, describes compositions containing ca. 97.5% w/w of kaolin, said amount lies outside the range of present claim 1.

WO 00/075079 relates to an effervescent therapeutic composition containing a clay useful for internal or external use. The composition comprises between 40 to 85% of single clay minerals, together with effervescent ingredients.

DE 20009867 U1 relates to the composition as described in WO 00/075079.

Whilst therapeutic compositions comprising clays are known in the art, there nevertheless remains a need for improved therapeutic clay compositions. It is the aim of the present invention to provide such an improved composition. A composition according to the present invention will comprise one or more of the following advantages:
(a) is palatable and has improved organoleptic properties e.g. improved smooth taste and mouthfeel;
(b) is less messy and easier to handle than commercially available powder preparations;
(c) does not have the problem of foaming associated with the use of effervescent agents;
(d) when in the form of a tablet, provides the required dose in one tablet so that only one daily dose is required; and
(e) has an improved (shorter) disintegration time.

According to the present invention there is provided a therapeutic composition comprising kaolin, at least one disintegrant, at least one lubricant and at least one binding agent, as described in claim 1.

In one aspect, a therapeutic composition according to the present invention is a composition for oral administration, which may be formulated so as to be palatable. In one aspect, the composition of the present invention is for internal cleansing of the body.

In another aspect, a therapeutic composition according to the present invention is for external application to the body. In one aspect, the composition of the present invention is for external cleansing of the body.

In another aspect, a therapeutic composition according to the present invention is for internal or external cleansing of the body.

In one aspect, a composition according to the present invention is in the form of a tablet. In a further aspect, a tablet for oral administration is a swallow tablet or a tablet to be chewed. In another aspect, the composition is a non-effervescent composition, for example a non-effervescent tablet.

A composition of the present invention may be dispersed in a liquid prior to ingestion or prior to external administration. For example, the composition may be dispersed in a suitable amount of water for oral consumption. In an alternative embodiment, the composition may be mixed with water to form a paste for external application.

A compositions according to the present invention comprises kaolin of a pharmaceutically acceptable grade, as may be obtained, for example, from Merck Pharmaceuticals or from Amberger Kaolin Werke. Kaolin for use in a composition of the present invention may be heavy or light kaolin.

In one aspect, the at least one clay, such as kaolin, for use in the invention, is present in the composition in an amount of from 30.0 to 70.0 % w/w, for example from 40.0 to 60.0 % w/w.

A composition according to the invention comprises at least one disintegrant. Suitable disintegrants for use in the invention include commercially available disintegrants commonly used in oral dosage forms. In one aspect, the at least one disintegrant is selected from: sodium starch glycolate, crosslinked povidone, calcium carboxymethylcellulose and crosslinked carboxymethylcellulose, or is a combination of two or more thereof.

In one aspect, the at least one disintegrant is present in the composition in an amount of from 1.0 to 8.0 % w/w, for example from 2.0 to 5.0% w/w.

A composition according to the invention comprises at least one lubricant. Suitable lubricants for use in the invention include commercially available lubricants commonly used in oral dosage forms. In one aspect, the at least one lubricant is selected from: fumaric acid, magnesium lauryl sulphate, solid polyethylene glycol (PEG), sodium lauryl sulphate, calcium behenate, calcium arachinate and sodium stearyl sulphate, or is a combination of two or more thereof.

In one aspect, the at least one lubricant is present in the composition in an amount of from 1.0 to 8.0 % w/w, for example from 2.0 to 5.0 % w/w.

A composition according to the invention comprises at least one binding agent. Suitable binding agents for use in the invention include commercially available binding agents commonly used in oral dosage forms. In one aspect, the at least one binding agent is selected from: microcrystalline cellulose, sorbitol, mannitol, polyvinylpyrrolidone (PVP), calcium phosphate, saccharose (sucrose) and lactose, or is a combination of two or more thereof.

In one aspect, the at least one binding agent is present in a composition in an amount of from 1.0 to 40.0 % w/w, for example from 5.0 to 40.0 % w/w, or from 5.0 to 30.0 % w/w.

A composition of the invention optionally further comprises a colouring agent which may be commercially available. In one aspect the optional colouring agent is selected from: red iron oxide, yellow iron oxide and a combination thereof.

In one aspect, the colouring agent may be present in a composition in an amount of from 1.0 to 5.0% w/w, for example from 2.0 to 4.0% w/w.

A composition of the invention optionally further comprises ingredients typically used in oral dosage forms such as a flavouring agent, compression aid; preservative; wetting agent; bulking agent; adhesive or a sweetening agent.

The invention also provides a process for the preparation of a composition of the invention, which process comprises admixing at least one clay, at least one disintegrant, at least one lubricant, at least one binding agent, and optionally any other desired ingredient, to form a mixture or blend, using techniques commonly used in the field, and optionally tableting the mixture or blend.

When a composition is in the form of a tablet, the tablet is compressed to provide a hardness of greater than about 100N, for example from about 100 to about 140N. It has been found that a tablet hardness in the range of from about 100 to about 140N facilitates rapid disintegration and ease of packaging and handling of the tablet.

Tableting may be achieved by techniques commonly used in the field of tablet making.

In one aspect, a composition according to the invention as herein described undergoes rapid disintegration in the presence of water. A composition, for example when in the form of a tablet, disintegrates in water within about 15 minutes or less prior to administration.

In one aspect, a composition of the present invention has a disintegration time in water of about 5 to about 15 minutes, for example of about 8 to about 12 minutes.

In one aspect, a tablet composition according to the present invention comprises a therapeutic dose of from about 2 to about 5 g of clay. In one aspect, a tablet comprising such a dose will comprise one or more score lines, i.e. break lines or grooves, to enable ease of breaking of the tablet, as may be necessary for ease of dosing, in particular if the tablet is of a large size. In a further aspect, the tablet comprises a plurality of score lines. Score lines are typically introduced during compression of the powder mixture by means of a suitably shaped punch (mould).

### Examples

The following Examples are illustrative of the invention.

### Example 1. The following tablet formulation according to the present invention was prepared.

| **Ingredient** | **Function** | **Amount mg/tablet** | **Percentage w/w** |
|---|---|---|---|
| Kaolin (heavy) | Active ingredient | 3000 | 50 % |
| Microcrystalline cellulose | Binder | 1200 | 20 % |
| Sorbitol | Binder/Flavor | 780 | 13 % |
| Mannitol | Binder/Flavor | 420 | 7 % |
| Calcium arachinate/behenate | Lubricant | 300 | 5 % |
| Sodium starch glycolate | Disintegrant | 180 | 3% |
| Iron oxide, red | Colour | 120 | 2 % |

Kaolin, microcrystalline cellulose, mannitol, sorbitol and iron oxide were roughly weighed and sieved. These components (kaolin, microcrystalline cellulose, mannitol, sorbitol and iron oxide) were weighed accurately into a container and blended together in a container mixer. 1/3 of the total amount of calcium arachinate/behenate was sieved and added to the mixture and a second blending step was carried out. Subsequently, the powder mixture was compressed in a dry granulation step using a roller-compactor. The resulting ribbons were granulated with a granulation unit connected to the roller compactor. The remaining 2/3 of the total amount of calcium arachinate/behenate, and sodium starch glycolate were roughly weighed, sieved, added to the granulate and mixed in a container mixer.

The powder mixture was compressed to tablets on a rotary tablet press.

### Examples 2-5: The following tablet formulations according to the present invention can be prepared as follows.

The active ingredient, the binders and the colouring agent are roughly weighed and sieved. These components (active ingredient, binders and colouring) are weighed accurately into a container and blended in a container mixer. 1/3 of the total amount of lubricant is sieved and added to the mixture and a second blending step is carried out. Subsequently, the powder mixture is compressed in a dry granulation step using a roller-compactor. The resulting ribbons are granulated with a granulation unit connected to the roller compactor. The remaining 2/3 of the total amount of lubricant and the disintegrant are roughly weighed, sieved, added to the granulate and mixed in a container mixer.

The powder mixture is compressed to tablets on a rotary tablet press.

### Example 2.

| **Ingredient** | **Function** | **Amount mg/tablet** | **Percentage w/w** |
|---|---|---|---|
| Kaolin (heavy) | Active Ingredient | 2700 | 45 % |
| Microcrystalline cellulose | Binder | 1200 | 20 % |
| Povidone | Binder | 300 | 5 % |
| Sorbitol | Binding agent/Flavour | 600 | 10 % |
| Mannitol | Binding agent/Flavour | 600 | 10 % |
| Calcium arachinate/behenate | Lubricant | 300 | 5 % |
| Povidone crosslinked | Disintegrant | 180 | 3 % |
| Iron oxide | Colour | 120 | 2 % |

### Example 3.

| **Ingredient** | **Function** | **Amount mg/tablet** | **Percentage** |
|---|---|---|---|
| Kaolin (heavy) | Active ingredient | 3000 | 50 % |
| Microcrystalline cellulose | Binder | 1200 | 20 % |
| Sucrose | Binder/Flavor | 1200 | 20 % |
| Calcium arachinate/behenate | Lubricant | 300 | 5 % |
| Sodium starch glycolate | Disintegrant | 180 | 3% |
| Iron oxide, red | Colour | 120 | 2 % |

### Example 4.

| **Ingredient** | **Function** | **Amount mg/tablet** | **Percentage** |
|---|---|---|---|
| Kaolin (heavy) | Active ingredient | 3000 | 50 % |
| Microcrystalline cellulose | Binder | 1800 | 30 % |
| Sucrose | Binder/Flavor | 600 | 10 % |
| Calcium arachinate/behenate | Lubricant | 300 | 5 % |
| Sodium starch glycolate | Disintegrant | 180 | 3% |
| Iron oxide, red | Colour | 120 | 2 % |

### Example 5.

| **Ingredient** | **Function** | **Amount mg/tablet** | **Percentage** |
|---|---|---|---|
| Kaolin (heavy) | Active ingredient | 3000 | 50 % |
| Microcrystalline cellulose | Binder | 1200 | 20 % |
| Sucrose | Binder/Flavor | 600 | 10 % |
| Lactose | Binder | 600 | 10 % |
| Calcium arachinate/behenate | Lubricant | 300 | 5 % |
| Sodium starch glycolate | Disintegrant | 180 | 3% |
| Iron oxide, red | Colour | 120 | 2 % |

### Example 6. Measurement of disintegration time

Tablet disintegration may be measured with the disintegration apparatus and testing method described in Ph. Eur. 01/2006; 2.9.1. "Disintegration of tablets and capsules". The following is an Example of the process used to measure disintegration time of the compositions of the present invention:

Three tablets were employed and placed one each into the three cylindrical transparent tubes of the bigger basket-rack assembly of the disintegration tester (type: Erweka ZT32). The tablets were covered with the respective plastic discs. The water bath of the apparatus was heated to a temperature of 36 C. From time to time the apparatus was stopped to monitor the disintegration process, because the disintegration medium (water) became cloudy while the tablets disintegrated. The disks covering the tablets were lifted by means of tweezers to enable a visual control of the disintegration process and the status of disintegration respectively. The time needed to disintegrate was determined using a clock.

The disintegration time is defined as: the time elapsed when all three tablets are completely disintegrate and no residue is observed in the tubes of the basket.

The disintegration time of Example 1 was measured according to the above procedure. Example I was found to have a disintegration time of 10 minutes.

### Example 7. Measurement of tablet hardness

Tablet hardness may be defined as the resistance to crushing of tablets, measured by the force needed to disrupt them by crushing

Tablet hardness may be measured with the disintegration apparatus and testing method described in Ph. Eur. 01/2008; 2.9.8. "Resistance to crushing of tablets". The following is an Example of the process used to measure tablet hardness of the compositions of the present invention:
Five tablets were tested using the high speed hardness tester HDT 1 V.3 (made by, and purchased from CGS GmbH). The tablets were placed between the jaws and subsequently the "Test for crushing strength" was chosen from the menu of the DOS-based software QPBT1.EXE (also made by, and purchased from CGS GmbH).

The tablet hardness in Newtons (N) is calculated as the mean value obtained for the five tablets, and is expressed as a range, (taking into account a relative standard deviation of 5-10%).

The tablet hardness of Example 1 was measured according to the above procedure. Example 1 was found to have a tablet hardness of 100-130 N.

## Claims

1. A therapeutic composition comprising kaolin present in an amount of from 30.0 to 70.0 % w/w, at least one disintegrant present in an amount of from 1.0 to 8.0 % w/w., at least one lubricant present in an amount of from 1.0 to 8.0% w/w and at least one binding agent present in an amount of from 1.0 to 40.0 % w/w.

2. A therapeutic composition according to claim 1 which is in the form of a tablet.

3. A therapeutic composition according to any one of claims 1 or 2, wherein the at least one disintegrant is selected from: sodium starch glycolate, crosslinked povidone, calcium carboxymethylcellulose and crosslinked carboxymethylcellulose, or is a combination of two or more thereof .

4. A therapeutic composition according to any one of claims 1 to 3, wherein the at least one lubricant is selected from: fumaric acid, magnesium lauryl sulphate, solid polyethylene glycol, sodium lauryl sulphate, calcium behenate, calcium arachinate and sodium stearyl sulphate, or is a combination of two or more thereof.

5. A therapeutic composition according to any one of claims 1 to 4, wherein the at least one binding agent is selected from: microcrystalline cellulose, sorbitol, mannitol, polyvinylpyrrolidone (PVP), calcium phosphate, saccharose (sucrose) and lactose, or is a combination of two or more thereof.

6. A therapeutic composition according to any one of claims 1 to 5 in the form of a tablet, wherein the tablet comprises a hardness of greater than about 100N.

7. A therapeutic composition according to any one of claims 1 to 6 for oral administration.

8. A therapeutic composition according to any one of claims 1 to 7 for internal or external cleansing of the body.

9. A therapeutic composition according to any one of claims 1 to 8, wherein the disintegration time of the composition in water is about 15 minutes or less.

10. Use of a therapeutic composition according to any of claims 1 to 9 for internal or external cleansing of the body.

## Patentansprüche

1. Eine therapeutische Zusammensetzung, umfassend Kaolin, das in einer Menge von 30,0 bis 70,0 % Gew./Gew. vorhanden ist, mindestens ein Sprengmittel, das in einer Menge von 1,0 bis 8,0 % Gew./Gew. vorhanden ist, mindestens ein Schmiermittel, das in einer Menge von 1,0 bis 8,0 % Gew./Gew. vorhanden ist, und mindestens ein Bindemittel, das in einer Menge von 1,0 bis 40,0 % Gew./Gew. vorhanden ist.

2. Eine therapeutische Zusammensetzung gemäß Anspruch 1, welche in Form einer Tablette vorliegt.

3. Eine therapeutische Zusammensetzung gemäß einem der Ansprüche 1 oder 2, wobei das mindestens eine Sprengmittel ausgewählt ist aus Natriumstärkeglycolat, vernetztem Povidon, Calciumcarboxymethylcellulose und vernetzter Carboxymethylcellulose oder eine Kombination von zwei oder mehreren davon ist.

4. Eine therapeutische Zusammensetzung gemäß einem der Ansprüche 1 bis 3, wobei das mindestens eine Schmiermittel ausgewählt ist aus Fumarsäure, Magnesiumlaurylsulfat, festem Polyethylenglycol, Natriumlaurylsulfat, Calciumbehenat, Calciumarachinat und Natriumstearylsulfat oder eine Kombination von zwei oder mehreren davon ist.

5. Eine therapeutische Zusammensetzung gemäß einem der Ansprüche 1 bis 4, wobei das mindestens eine Bindemittel ausgewählt ist aus mikrokristalliner Cellulose, Sorbitol, Mannitol, Polyvinylpyrrolidon (PVP), Calciumphosphat, Saccharose (Sucrose) und Lactose oder eine Kombination von zwei oder mehreren davon ist.

6. Eine therapeutische Zusammensetzung gemäß einem der Ansprüche 1 bis 5 in Form einer Tablette, wobei die Tablette ein Härte von mehr als etwa 100N umfasst.

7. Eine therapeutische Zusammensetzung gemäß einem der Ansprüche 1 bis 6 für die orale Verabreichung.

8. Eine therapeutische Zusammensetzung gemäß einem der Ansprüche 1 bis 7 für die innere oder äußere Reinigung des Körpers.

9. Eine therapeutische Zusammensetzung gemäß einem der Ansprüche 1 bis 8, wobei die Zerfallszeit der Zusammensetzung in Wasser etwa 15 Minuten oder weniger beträgt.

10. Verwendung einer therapeutischen Zusammensetzung gemäß einem der Ansprüche 1 bis 9 für die innere oder äußere Reinigung des Körpers.

## Revendications

1. Composition thérapeutique comprenant du kaolin présent en une quantité de 30,0 à 70,0 % en poids/poids, au moins un agent de délitement présent en une quantité de 1,0 à 8,0 % en poids/poids, au moins un lubrifiant présent en une quantité de 1, 0 à 8, 0 % en poids/poids et au moins un liant présent en une quantité de 1,0 à 40,0 % en poids/poids.

2. Composition thérapeutique suivant la revendication 1, qui est sous forme de comprimés.

3. Composition thérapeutique suivant l'une quelconque des revendications 1 et 2, dans laquelle ledit au moins un agent de délitement est choisi entre : le glycolate d'amidon sodique, la povidone réticulée, la carboxyméthylcellulose calcique et la carboxyméthylcellulose réticulée, ou représente une association de deux ou plus de deux de ces agents.

4. Composition thérapeutique suivant l'une quelconque des revendications 1 à 3, dans laquelle ledit au moins un lubrifiant est choisi entre : l'acide fumarique, le laurylsulfate de magnésium, le polyéthylèneglycol solide, le laurylsulfate de sodium, le béhénate de calcium, l'arachinate de calcium et le stéarylsulfate de sodium, ou bien est une association de deux ou plus de deux de ces lubrifiants.

5. Composition thérapeutique suivant l'une quelconque des revendications 1 à 4, dans laquelle ledit au moins un liant est choisi entre : la cellulose microcristalline, le sorbitol, le mannitol, la polyvinylpyrrolidone (PVP), le phosphate de calcium, le saccharose (sucrose) et le lactose, ou bien est une association de deux ou plus de deux de ces agents.

6. Composition thérapeutique suivant l'une quelconque des revendications 1 à 5, sous forme de comprimés, dans laquelle le comprimé a une dureté supérieure à environ 100 N.

7. Composition thérapeutique suivant l'une quelconque des revendications 1 à 6, pour administration orale.

8. Composition thérapeutique suivant l'une quelconque des revendications 1 à 7, pour l'épuration interne ou externe de l'organisme.

9. Composition thérapeutique suivant l'une quelconque des revendications 1 à 8, le temps de délitement de la composition dans l'eau étant approximativement égal ou inférieur à 15 minutes.

10. Utilisation d'une composition thérapeutique suivant l'une quelconque des revendications 1 à 9, pour l'épuration interne ou externe de l'organisme.
